# EUROPÄISCHE PATENTSCHRIFT

(11) **EP 2 670 729 B1**
(45) Veröffentlichungstag und Bekanntmachung des Hinweises auf die Patenterteilung: **22.07.2015**
(21) Anmeldenummer: 12703028.6
(22) Anmeldetag: 30.01.2012
(51) Int. Cl.: B01J 39/02, B01J 39/04, B01J 39/20, B01J 49/00, B01J 39/10, C07C 201/16

(54) **VERHINDERUNG VON AUSFÄLLUNGEN AUS NITRIERTEN AROMATISCHEN ROHPRODUKTEN**
PREVENTION OF PRECIPITATION FROM NITRATED AROMATIC CRUDE PRODUCTS
EMPÊCHEMENT DE PRÉCIPITATIONS À PARTIR DE PRODUITS BRUTS AROMATIQUES NITRIFIÉS

(30) Priorität: 31.01.2011 EP 11152741
(43) Veröffentlichungstag der Anmeldung: 11.12.2013
(73) Patentinhaber: BASF SE, 67056 Ludwigshafen (DE)
(72) Erfinder: ALLARDT, Holger, 01987 Schwarzheide (DE); RAICHLE, Andreas, 67063 Ludwigshafen (DE); REETZ, Reiner, 01987 Schwarzheide (DE); BÜTTNER, Johannes, 01945 Ruhland (DE); ZÖLLINGER, Michael, 73054 Eislingen (DE); HAASE, Stefanie, 01900 Bretnig-Hauswalde (DE); FRITZ, Rüdiger, 02994 Bernsdorf (DE)
(86) Internationale Anmeldenummer: PCT/EP2012/051457
(87) Internationale Veröffentlichungsnummer: WO 2012/104254

(56) Entgegenhaltungen:
- DE-B- 1 221 994
- GB-A- 1 031 450

## Beschreibung

Die vorliegende Erfindung betrifft ein Verfahren zur Verhinderung von Ausfällungen von Nitrohydroxyaromat-Salzen aus den bei der Nitrierung von aromatischen Verbindungen nach alkalischer Wäsche anfallenden nitrierten Rohprodukten sowie eine Produktionsanlage zur Nitrierung nitrierbarer aromatischer Verbindungen.

Die Nitrierung aromatischer Verbindungen ist von großer technischer Bedeutung, da die aromatische Nitrogruppe leicht in andere funktionelle Gruppen umgewandelt werden kann. Die Nitrierung aromatischer Verbindungen wird daher vielfältig eingesetzt, z. B. bei der Herstellung von Explosivstoffen, in der pharmazeutischen Industrie, bei der Herstellung von Kunststoffen, bei der Produktion von Farbstoffen und dergleichen. Das einfachste und technisch am häufigsten eingesetzte Nitrierungsreagenz sind Salpetersäure/Schwefelsäure-Gemische (so genannte "Nitriersäure") mit variablen Mengenverhältnissen der beiden Säuren. Dabei bewirkt die konzentrierte Schwefelsäure die Bildung des eigentlich wirksamen Nitrylkations und bindet daneben das beim Nitrierprozess entstehende Wasser.

Beispielsweise wird Mononitrotoluol (MNT) durch Nitrierung aus Toluol hergestellt. Das nach Abtrennung der Nitriersäure anfallende Roh-MNT enthält noch Nitrokresole, Salpetersäure, Stickstoffoxide und andere Abbauprodukte. Das Roh-MNT wird durch Wäsche mit Wasser in mehreren Stufen von den vorgenannten Verunreinigungen befreit. In einer ersten Stufe (saure Wäsche) werden mit Wasser alle starken Säuren, wie Schwefelsäure, Salpetersäure und Salpetrige Säure bzw. Stickstoffoxide, ausgewaschen. In einer zweiten Waschstufe (Alkaliwäsche) werden mit einer wässrigen Lösung einer Base, wie Natriumcarbonat-Lösung oder Natronlauge, alle schwach sauren Stoffe, wie z. B. Nitrokresole, Pikrinsäure und Nitrobenzoesäuren, ausgewaschen. In einer letzten (neutralen) Waschstufe mit Wasser können weitere Mengen an Verunreinigungen entfernt werden.

Es hat sich gezeigt, dass die Extraktion der Nitrohydroxyaromaten, wie Nitrokresolen, in die wässrige Phase nicht quantitativ erfolgt. Ein Teil der Nitrokresole verbleibt als Nitrokresolate in den Nitroaromaten gelöst. In der organischen Phase gelöstes oder dispergiertes Wasser verstärkt diesen Effekt. Bei der Aufreinigung der Nitroaromaten, z. B. durch Destillation, bzw. bei der Lagerung, kann es zum Auskristallisieren der Nitrokresolate kommen. Bei der Destillation kommt es zunächst zu einer Aufkonzentrierung der Nitrokresolate, die dann an Behälterwänden und Rohrleitungen auskristallisieren können. Auch beim längeren Lagern können die Nitrokresolate auskristallisieren. Da es sich bei Nitrokresolaten um instabile und in reiner Form explosive Verbindungen handelt, kann es durch mechanische oder thermische Stimulation zu einer unerwünschten Explosion kommen. Derartige Explosionen können auch als eine Initialzündung dienen und zu einer Detonation der damit in Kontakt stehenden Hauptmenge an Nitroaromaten führen.

Ähnliche Probleme, wie sie vorstehend im Zusammenhang mit der Nitrierung von Toluolen beschrieben wurden, können auch bei der Nitrierung anderer nitrierbarer Aromaten mit Nitriersäure auftreten, z. B. bei der Herstellung von Nitrobenzolen. Nitrophenole oder Nitrophenolate können sich bei der Destillation aufkonzentrieren und an Behälterwänden und Rohrleitungen auskristallisieren.

GB-A-1 031 450 offenbart ein Verfahren zur Entfernung von Nitrophenolen. Dabei werden die nitrierten Rohprodukte mit Wasser gewaschen, anschließend mit einer verdünnten, wäßrigen Natriumhydroxid-Lösung vermischt und dieses Gemisch dann über einen basischen Anionenaustauscher geführt. Der Erfindung liegt die Aufgabe zugrunde, ein Verfahren zur Verhinderung von potentiell gefährlichen Ausfällungen aus den bei der Nitrierung von aromatischen Verbindungen nach alkalischer Wäsche anfallenden nitrierten Rohprodukten anzugeben.

Die Aufgabe wird gelöst durch ein Verfahren zur Verhinderung von Ausfällungen von Nitrohydroxyaromat-Salzen aus den bei der Nitrierung von aromatischen Verbindungen nach alkalischer Wäsche anfallenden nitrierten Rohprodukten, das dadurch gekennzeichnet ist, dass man die nitrierten Rohprodukte in Kontakt mit einem sauren Ionentauscher bringt.

Im Rahmen der vorliegenden Erfindung werden unter dem Begriff der nitrierbaren aromatischen Verbindungen insbesondere Benzol, Toluol, Xylole, Chlorbenzol, Dichlorbenzole, Mononitrobenzol, Mononitrotoluole, Mononitrochlorbenzol, Dinitrotoluole etc. verstanden. Benzol und Toluol sind bevorzugt.

Die Nitrierung erfolgt in an sich bekannter Weise. Sie wird bevorzugt bei Temperaturen von 30 bis 100 °C, insbesondere 30 bis 70 °C und ganz besonders bevorzugt bei 35 bis 50 °C durchgeführt. Als Nitriersäure wird eine üblicherweise bei der Nitrierung von Aromaten verwendete Nitriersäure eingesetzt. Die Nitriersäure, ein Gemisch aus Schwefel- und Salpetersäure, besitzt im allgemeinen einen Salpetersäuregehalt von 0,5 bis 40, bevorzugt 25 bis 36 Gew.-%. Das Molverhältnis von nitrierbaren aromatischen Verbindungen zu Salpetersäure beträgt bevorzugt 1:1 bis 1:1,3, besonders bevorzugt 1:1,02 bis 1:1,2.

Nach Abtrennung der Nitriersäurephase (d. h. der sauren wässrigen Phase) werden die nitrierten Rohprodukte einer Wäsche mit Wasser unterzogen. Die Wäsche kann als ein- oder mehrstufiges Extraktionsverfahren ausgestaltet sein, die als Flüssig/Flüssig-Extraktion durchgeführt wird. Das Extraktionsverfahren kann z. B. eine Querstromextraktion, eine Gegenstromextraktion oder Kombinationen davon umfassen.

Die nitrierten Rohprodukte werden anschließend einer weiteren Aufreinigung unterzogen. Diese erfolgt mittels alkalischer und gegebenenfalls nachfolgender neutraler Wäsche, welcher sich wiederum gegebenenfalls eine Trocknung der nitrierten Produkte anschließen kann.

Als Base in der alkalischen Wäsche kommen wässrige Lösungen oder Aufschlämmungen von Alkalimetall(hydrogen)carbonaten, Alkalimetallhydroxiden, Erdalkalimetall(hydrogen)carbonaten oder Erdalkalimetallhydroxiden, wie Natriumcarbonat, Natriumhydrogencarbonat, Natronlauge oder Kalkmilch, in Betracht. Wässrige Lösungen von Natriumcarbonat sind im Allgemeinen bevorzugt.

Bei der Nitrierung werden typischerweise Gemische von nitrierten aromatischen Verbindungen mit unterschiedlichem Nitriergrad erhalten, d. h. Gemische einfach, zweifach und/oder mehrfach nitrierter aromatischer Verbindungen. Zeigen die nitrierten Vertreter Regioisomerie, werden im Allgemeinen Gemische von Regioisomeren erhalten.

In einer bevorzugten Ausführungsform enthalten die nitrierten Rohprodukte wenigstens 85 Gew.-% Mononitrotoluole.

Eine typische Zusammensetzung Roh-MNT (technisches MNT), wie es durch Nitrierung von Toluol und anschließende alkalische Wäsche hergestellt werden kann, lautet:
56 bis 61 Gew.-%, häufig 58,5 bis 59,5 Gew.-% o-Nitrotoluol (NT),
4,0 bis 4,5 Gew.-%, häufig 4,1 bis 4,3 Gew.-% m-NT,
35 bis 38 Gew.-%, häufig 36,0 bis 36,5 Gew.-% p-NT,
0,3 bis 0,8 Gew.-%, häufig 0,4 bis 0,7 Gew.-% Dininitrotoluol (DNT),
0,02 bis 0,5 Gew.-%, häufig 0,05 bis 0,4 Gew.-% Wasser,
0,00001 bis 0,2 Gew.-% Toluol,
0,0001 bis 0,05 Gew.-%, häufig 0,0001 bis 0,02 Gew.-% unterschiedlicher Isomere an Mono-, Di und Trinitrokresolaten sowie
0 bis 0,01 Gew.-% Trinitrotoluol (TNT).

Abhängig von den pH-Werten bei den Wäschen können auch Mono-, Di- und Trinitrokresole in Spuren enthalten sein.

Die nitrierten Rohprodukte werden üblicherweise durch Destillation nach ihrem Siedepunkt aufgetrennt. Die Auftrennung und Reinigung erfolgt im Allgemeinen durch einoder mehrstufige Destillation in üblichen Rektifizierkolonnen. Zweckmäßigerweise wird der zu trennende Zulauf an einer zwischen dem Kolonnenkopf und Kolonnensumpf gelegenen Stelle seitlich in die Kolonne eingeführt. Ein gewünschtes Wertprodukt kann, je nach relativer Lage der Siedepunkte der zu trennenden Komponente und der Reinheitsanforderungen, z. B. als Kopfprodukt, Seitenabzug im Verstärkungsteil, Seitenabzug im Abtriebsteil oder Sumpfprodukt gewonnen werden.

Die Kolonne(n) sind mit einer Sumpfheizung versehen, die innenliegend oder vorzugsweise außenliegend mit Naturumlauf oder vorzugsweise Zwangsumlauf (über eine Pumpe) ausgestaltet sein kann.

Eine mögliche Auftrennung der nitrierten Rohprodukte ist nachstehend am Beispiel eines Roh-MNT dargestellt. Das Roh-MNT kann seitlich in eine o-Nitrotoluol-Kolonne eingespeist werden, über deren Kopf Leichtsieder abgezogen werden. An geeigneter Stelle im Verstärkungsteil wird das Wertprodukt o-NT flüssig im Seitenabzug entnommen. Das Sumpfprodukt der o-Nitrotoluol-Kolonne, das ein Gemisch aus p-NT, m-NT und DNT umfasst, wird seitlich in eine zweite Kolonne eingespeist, in deren Sumpf ein Gemisch von m-NT, p-NT und DNT anfällt. Das Kopfprodukt der zweiten Kolonne enthält einen Hauptanteil an p-NT und m-NT. Kondensiertes Kopfprodukt wird seitlich in eine dritte Kolonne eingespeist, in der ein Kopfprodukt aus m-NT und p-NT und reines p-NT als Sumpfprodukt anfällt.

Nach destillativer Abtrennung von o-NT in der o-Nitrotoluol-Kolonne ergibt sich typischerweise ein Sumpfprodukt der Zusammensetzung:
0,2 bis 1 Gew.-% o-NT,
7 bis 13 Gew.-% m-NT,
86 bis 90 Gew.-% p-NT,
1 bis 2 Gew.-% DNT,
0,0002 bis 0,5 Gew.-%, häufig 0,001 bis 0,03 Gew.-% unterschiedlicher Isomere an Mono-, Di und Trinitrokresolaten sowie
jeweils 0 bis 0,01 Gew.-% Toluol, TNT und Wasser.

Abhängig von den pH-Werten bei den Wäschen können auch Mono-, Di- und Trinitrokresole in Spuren enthalten sein.

Nach destillativer Abtrennung eines m-NT und p-NT reichen Produktstroms in der MNT-Kolonne ergibt sich dort typischerweise ein Sumpfprodukt der Zusammensetzung:
0,0001 bis 0,1 Gew.-% o-NT,
2 bis 4 Gew.-% m-NT,
82 bis 87 Gew.-% p-NT,
10 bis 13 Gew.-% DNT,
0,001 bis 10 Gew.-%, häufig 0,005 bis 1 Gew.-%, meist 0,01 bis 0,05 Gew.-% unterschiedlicher Isomere an Mono-, Di und Trinitrokresolaten sowie
jeweils 0 bis 0,01 Gew.-% Toluol, TNT und Wasser.

Abhängig von den pH-Werten bei den Wäschen können auch Mono-, Di- und Trinitrokresole in Spuren enthalten sein.

Erfindungsgemäß bringt man die nitrierten Rohprodukte in Kontakt mit einem sauren Ionentauscher. Vorzugsweise handelt es sich um einen Stoffstrom von nitrierten Rohprodukten, der ausgewählt ist unter einem Zulauf zu einer Destillationskolonne, einem Sumpfumlauf einer Destillationskolonne und einem Zulauf zu einem Verdampfer. Die Anordnung der Ionentauschereinheit im Sumpfumlauf ist nicht kritisch; die Ionentauschereinheit kann zwischen Kolonnensumpf und Pumpe, zwischen Pumpe und Aufheizer oder zwischen Aufheizer und Umlaufaufgabe angeordnet sein.

Die nitrierten Rohprodukte enthalten vor der erfindungsgemäßen Behandlung mit einem sauren Ionenaustauscher im Allgemeinen 0,0001 bis 0,05 Gew.-%, meist 0,0001 bis 0,02 Gew.-%, Nitrohydroxyaromat-Salze.

Durch die erfindungsgemäße Behandlung werden Nitrohydroxyaromat-Salze, die im Rahmen der Flüssig/Flüssig-Extraktion in den nitrierten Rohprodukten verblieben sind, in die Säureform überführt, die ausreichende Löslichkeit in den nitrierten Rohprodukten zeigen und bei der Destillation und/oder Lagerung nicht auskristallisieren.

Der Erfolg der erfindungsgemäßen Behandlung kann überwacht bzw. überprüft werden, indem man den Gehalt der behandelten Stoffströme an Alkali- bzw. Erdalkaliionen bestimmt. Der Gehalt an Alkali- und Erdalkalüonen in den behandelten Rohprodukten sollte weniger als 0,1 mg/L betragen.

Alternativ ist eine spektroskopische Bestimmung des Gehalts an Nitrohydroxyaromat-Salzen möglich. Beispielsweise können VIS-spektroskopische Messungen in einem Wellenlängenbereich von 400 bis 600 nm (die maximale Absorptionswellenlänge von Natrium-2,6-dinitro-p-kresolat beträgt beispielsweise 464 nm) durchgeführt werden. Die Messung kann beispielsweise als Vergleichsmessung vor und nach der Ionentauschereinheit durchgeführt werden oder nach der Ionenaustauschereinheit nach zuvor erfolgter Kalibrierung bezüglich der Nitrohydroxyaromat-Salze.

Bei dem erfindungsgemäß verwendeten sauren Ionentauscher handelt es sich um einen Kationenaustauscher in Säureform.

Als Kationenaustauscher kommen grundsätzlich alle Ionenaustauschermaterialien, z. B. organische Ionenaustauscherharze oder anorganische Ionenaustauscher, in Betracht, die saure Gruppen, in der Regel Sulfonsäuregruppen, aufweisen. Häufig handelt es sich um partikelförmige, mäßig oder stark vernetzte organische Polymere, häufig auf der Basis von Polystyrol, die an der Oberfläche der Polymerpartikel eine Vielzahl saure Gruppen aufweisen. Die durchschnittliche Konzentration der sauren Gruppen liegt üblicherweise im Bereich von 1 bis 15 meq/kg lonentauscherharz. Die mittlere Teilchengröße der lonentauscherpartikel liegt typischerweise im Bereich von 0,1 bis 4 mm, wobei größere als auch kleinere Teilchengrößen je nach Abmessung der lonentauscheranordnung geeignet sein können. Die Polymerteilchen können beispielsweise gelartig sein oder eine makroporöse Struktur aufweisen.

Derartige Ionentauscher sind bekannt und werden kommerziell angeboten, beispielsweise unter den Handelsbezeichnungen Lewatit® K oder Lewatit® S der Fa. LAN-XESS, z.B. Lewatit® K 2629; Amberjet®, Amberlyst® oder Amberlite® der Fa. Rohm & Haas, z.B. Amberlyst® 35 dry; Dowex® der Firma Dow Chemicals, z.B. Dowex® UP-COR® Mono C-600; Diaion® der Fa. Mitsubishi Chemical Corp., z.B. Diaion® SK1B; weiterhin Zorbax® 300 SCX (Silica-basierter Kationenaustauscher) der Fa. Agilent Technologies Deutschland GmbH.

Das Inkontaktbringen mit dem Ionentauscher kann auf jede erdenkliche Art erfolgen. Man kann Ionenaustauscher in den nitrierten Rohprodukten verteilen und anschließend abtrennen, z. B. durch Dekantieren oder Filtrieren.

Vorzugsweise leitet man die nitrierten Rohprodukte über eine Schüttung eines sauren Ionentauschers. Der Ionentauscher liegt in einer Festbettschüttung vor, die in einer Säule angeordnet ist, durch die der Stoffstrom geleitet wird. Die Säule ist vorzugsweise vertikal angeordnet und wird vom Stoffstrom in Richtung der Schwerkraft oder entgegen der Schwerkraft durchströmt. Die Ausdehnung des Festbetts in Strömungsrichtung beträgt vorzugsweise das 2- bis 15-fache des (längsten) Durchmessers des Festbetts. Es können auch mehrere hintereinandergeschaltete Säulen verwendet werden.

Die spezifische Fließrate SV, d.h. das Verhältnis von durchschnittlicher Fließrate V (Volumengeschwindigkeit), mit der die nitrierten Rohprodukte durch die Ionenaustauscheranordnung geleitet werden, zum Gesamtvolumen des Ionenaustauschers in der Ionenaustauscheranordnung (Bettvolumen BV), ist von untergeordneter Bedeutung und liegt typischerweise im Bereich von 0,1 bis 5 h⁻¹.

Die Temperatur, bei der die Behandlung erfolgt, liegt oberhalb der Schmelztemperaturen der nitrierten Produkte, typischerweise im Bereich von 0 bis 150°C, vorzugsweise im Bereich von 20 bis 120°C.

Nach einer Betriebsdauer ist der Ionentauscher gesättigt. Der Ionentauscher kann durch Überleiten einer Säure, vorzugsweise Schwefelsäure, regeneriert werden. Nach der Regenerierung ist eine Spülung mit Wasser oder ammoniakalischem Wasser zweckmäßig. Bevorzugt erfolgt dies in einem Säure-/Abwasserverbund mit einer Nitrieranlage.

Weiterer Gegenstand der vorliegenden Erfindung ist eine Produktionsanlage zur Nitrierung nitrierbarer aromatischer Verbindungen, wobei die Produktionsanlage die folgenden Einheiten umfasst:
a) eine Nitriereinheit zur Nitrierung nitrierbarer aromatischer Verbindungen,
b) in Produktionslinie stromabwärts zur Nitriereinheit angeordnet, eine Einheit zur Durchführung einer sauren Wäsche mittels Extraktion,
c) in Produktionslinie stromabwärts zur Einheit zur Durchführung der sauren Wäsche angeordnet, eine Einheit zur Durchführung einer alkalischen Wäsche,
d) in Produktionslinie stromabwärts zur Einheit zur Durchführung der alkalischen Wäsche angeordnet, eine Einheit zur Durchführung einer neutralen Wäsche,
e) in Produktionslinie stromabwärts zur Einheit zur Durchführung der neutralen Wäsche angeordnet, eine Destillationseinheit mit einer oder mehreren Destillationskolonnen, und
f) im Zulauf zu wenigstens einer Destillationskolonne und/oder wenigstens einem Sumpfumlauf einer Destillationskolonne, wenigstens eine Ionentauschereinheit zum Inkontaktbringen nitrierter Rohprodukte mit einem sauren Ionentauscher.

Die Erfindung wird durch die beigefügte Zeichnung und die folgenden Beispiele näher veranschaulicht.
Fig. 1 zeigt eine zur Durchführung des erfindungsgemäßen Verfahrens geeignete Anlage bei der Isomerentrennung von Mononitrotoluol.
Fig. 2 zeigt eine Anordnung zur diskontinuierlichen Behandlung eines nitrierten Rohprodukts mit einem sauren Ionentauscher.
Fig. 3 zeigt eine Anordnung zur kontinuierlichen Behandlung eines nitrierten Rohprodukts mit zwei parallelen lonentauschereinheiten.

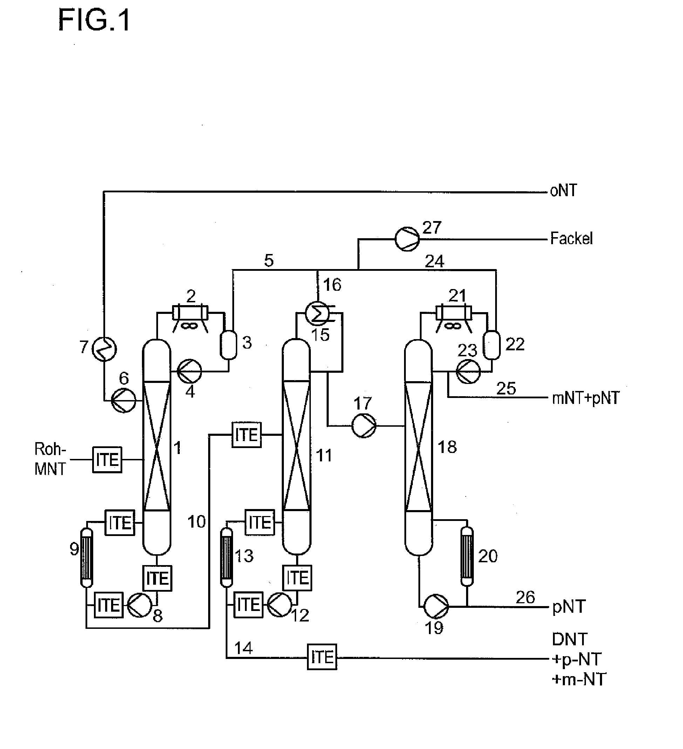

Mit Bezug auf Fig. 1 wird mittels einer Pumpe (nicht dargestellt) das gewaschene Roh-MNT mit einer Temperatur von etwa 65°C unterhalb der Mitte in die Kolonne 1 eingespeist. In der Kolonne 1 erfolgt eine destillative Trennung bei einem Kopfdruck von 200 mbar und einer Sumpftemperatur von 187-189°C. Das Vakuumsystem 27 erzeugt das erforderliche Destillationsvakuum. Über Kopf abgezogene Leichtsieder werden über einen Luftkühler 2 teilweise kondensiert. Ein Teil des Kondensats gelangt über einen Zwischenbehälter 3 und eine Pumpe 4 als Rücklauf zurück zum Kopf der Kolonne 1. Die unkondensierten Anteile werden über die Leitung 5 abgezogen und verbrannt. Unterhalb des Kolonnenkopfs der Kolonne 1 wird über einen Seitenabzug und die Pumpe 6 das Wertprodukt o-NT mit einer Temperatur von etwa 162 °C abgezogen, im Wärmeaustauscher 7 auf etwa 60°C abgekühlt und in einen o-NT-Lagerbehälter (nicht dargestellt) gepumpt.

Eine Sumpfumlaufpumpe 8 fördert das Sumpfprodukt der Kolonne 1, das im Wesentlichen aus einem Gemisch von p-NT, m-NT und DNT besteht, aus dem Sumpf der Kolonne 1 über einen Fallfilmverdampfer 9; der Energieeintrag erfolgt dort mittels abgehitztem 22 bar Dampf. Die Ausschleusung des Sumpfproduktes erfolgt mengengeregelt als Teilstrom von der Druckseite der Pumpe 8 über die Leitung 10 zur Kolonne 11.

Die Einspeisung des Sumpfproduktes in die Kolonne 11 erfolgt oberhalb der Mitte der Kolonne 11. Die Destillation erfolgt unter einem Kopfdruck von 13 mbar und bei einer Sumpftemperatur von 116-119°C. Im Sumpf der Kolonne 11 fällt ein Gemisch von m-NT, p-NT und DNT an. Der Wärmeeintrag erfolgt über den Kreislauf einer Sumpfumlaufpumpe 12 und einen Fallfilmverdampfer 13 (Anströmung von oben in der Prinzipskizze nicht dargestellt), der mit abgehitztem 3,5 bar Dampf betrieben wird. Die Menge des Sumpfabzugs über die Leitung 14 in einen Austragsbehälter (nicht dargestellt) wird standgeregelt. Dabei wird ein DNT-Gehalt von etwa 10-13% eingestellt. Das Kopfprodukt der Kolonne 11 mit dem Hauptanteil an p-NT und m-NT wird in einem Wärmeaustauscher 15 mit Warmwasser kondensiert. Die unkondensierbaren Anteile werden über die Leitung 16 abgezogen und verbrannt. Eine Teilmenge des Kondensats strömt zurück zum Kopf der Kolonne 11. Die Hauptmenge wird mittels Pumpe 17 abgezogen und in die Kolonne 18 eingespeist. Mit dem mengenüberwachten Kopfproduktabzug aus der Kolonne 11 in die Kolonne 18 erfolgt die Temperaturregelung im Bereich des Kopfes der Kolonne 11.

In der Kolonne 18 erfolgt die Einspeisung des aus der Kolonne 11 ausgeschleusten Kopfproduktes mengenkontrolliert unterhalb des oberen Drittels. Die destillative Trennung erfolgt unter einem Kopfdruck von 250 mbar. Die Sumpftemperatur beträgt etwa 187-190°C. Der Stand im Sumpf der Kolonne 18 wird durch Abzug eines Teilstroms des Produkts pNT auf der Druckseite der Sumpfumlaufpumpe 19 geregelt. Der größere Mengenstrom wird mit dieser Pumpe über einen Fallfilmverdampfer 20 mit abgehitztem 19,5 bar Dampf aufgeheizt und zur Aufheizung des Sumpfes der Kolonne 18 verwendet. Über die Leitung 26 wird das Wertprodukt p-NT abgeführt. Das Kopfprodukt, das im Wesentlichen aus einem Gemisch von m-NT und p-NT besteht, wird in einem Luftkühler 21 kondensiert, ein Teilstrom wird über einen Zwischenbehälter 22 und eine Pumpe 23 an den Kopf der Kolonne 18 zurückgeführt. Die unkondensierbaren Anteile werden über die Leitung 24 angezogen und verbrannt. Die über die Leitung 25 ausgeschleuste Isomerenmischung kann z.B. durch Kristallisation weiter aufgetrennt oder als Isomerenmischung, gegebenenfalls zusammen mit dem Sumpfprodukt der Kolonne 11, für die Herstellung von DNT eingesetzt werden.

An wenigstens einer der in der Fig. 1 mit ITE gekennzeichneten Positionen ist erfindungsgemäß eine Ionenaustauschereinheit vorgesehen, um den jeweiligen Stoffstrom mit einem sauren Ionenaustauscher in Kontakt zu bringen.

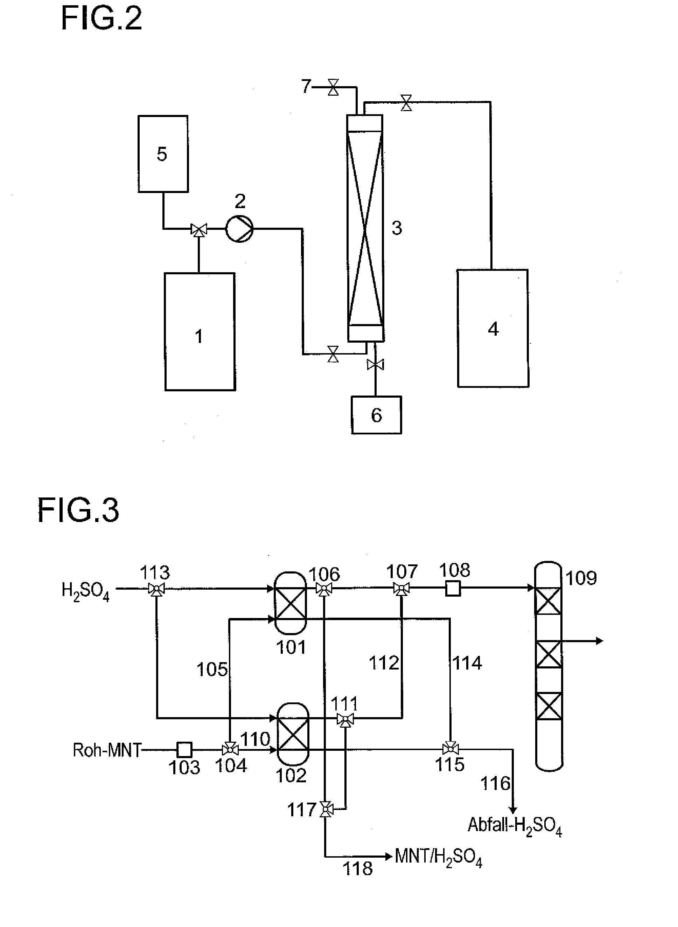

Mit Bezug auf Fig. 3 wird ein Roh-MNT-Strom über eine VIS-Messstelle 103 geleitet, wobei ein Referenzwert des Gehalts an Nitrokresolat bestimmt wird. Das Dreiwegeventil 104 ist so eingestellt, dass der Roh-MNT-Strom über die Leitung 105 zur lonentauschersäule 101 geführt wird und diese von unten nach oben durchströmt. Die Dreiwegeventile 106 und 107 sind so gestellt, dass der behandelte MNT-Strom über die VIS-Messstelle 108 zur Destillationskolonne 109 geführt wird. Durch Vergleich des in der Messstelle 108 gefundenen Messwertes mit dem Referenzwert des Gehalts an Nitrokresolat kann der Erfolgt der lonentauscherbehandlung überprüft werden. Sobald der Ionentauscher in der Säule 101 erschöpft ist, werden die Dreiwegeventile 104 und 107 umgelegt und der Roh-MNT-Strom fließt nun über das Dreiwegeventil 104 zur Ionentauschersäule 102, durchströmt diese von unten nach oben und strömt über die Dreiwegeventile 111 und 107 zur VIS-Messstelle 108 und Destillationskolonne 109. Die Säule 101 kann nun regeneriert werden, indem über das Dreiwegeventil 113 Schwefelsäure herangeführt wird. Verbrauchte Schwefelsäure kann über die Leitung 114, Dreiwegeventil 115 und Leitung 116 abgelassen werden. Nach beendeter Regeneration wird Schwefelsäure in der Säule 101 durch Roh-MNT verdrängt, das über die Leitung 105 herangeführt wird; die MNT/Schwefelsäure-Mischphase wird über das Dreiwegeventil 106, 117 und Leitung 118 abgeführt. In ähnlicher Weise kann die Ionentauschersäule 102 regeneriert werden, indem Schwefelsäure über das Dreiwegeventil 113 zur Säule 102 geführt und über das Dreiwegeventil 115 und Leitung 116 abgelassen wird. Nach beendeter Regeneration wird Schwefelsäure in der Säule 102 durch Roh-MNT verdrängt, das über die Leitung 110 herangeführt wird; die MNT/Schwefelsäure-Mischphase wird über die Dreiwegeventile 111, 117 und Leitung 118 abgeführt.

### Beispiele

### Beispiel 1:

In einem mit Stickstoff inertisierten 1000 mL Dreihalskolben wurden 500 g MNT (0,01 Gew.-% o-NT, 2,7 Gew.-% m-NT, 85,8 Gew.-% p-NT, 11,5 Gew.-% DNT, 0,023 Gew.-% unterschiedlicher Isomere an Mono-, Di- und Trinitrokresolaten sowie jeweils < 0,01 Gew.-% Toluol, TNT und Wasser) unter Rühren mit 0,1 Gew.-% Amberlyst® 35 dry (saurer Ionenaustauscher der Fa. Rohm & Haas) versetzt und drucklos bei 120°C gerührt. Um den Ionenaustausch verfolgen zu können, wurden in Abständen von 10 min Proben entnommen und mit Hilfe von ICP-AES auf den Gehalt an Natrium untersucht. Die Untersuchung ergab nach 40 min keine nachweisbaren Spuren von Natrium mehr. Die MNT Mischung verfärbte sich dabei von tiefrot nach gelblich klar. Nach dem Abfiltrieren wurde Na-freies MNT erhalten.

### Beispiel 2:

Es wurde genauso verfahren wie in Beispiel 1 mit der Ausnahme, dass 1 Gew.-% Ionenaustauscher eingesetzt wurden. Bereits nach 20 min war kein Natrium mehr nachweisbar.

### Beispiel 3:

Es wurde genauso verfahren wie in Beispiel 1 mit der Ausnahme, dass 5 Gew.-% Ionenaustauscher eingesetzt wurden. Bereits nach 10 min war kein Natrium mehr nachweisbar.

### Beispiel 4:

Es wurde genauso verfahren wie in Beispiel 1 mit der Ausnahme, dass DNT mit einem Nitrokresolat-Anteil von 300 ppm verwendet wurde. Nach 40 min war kein Natrium mehr nachweisbar.

### Beispiel 5:

Man verwendete eine Anordnung wie in Fig.2 dargestellt. In ein Glasrohr 3 mit dem Innendurchmesser von 5 cm und einer Länge von 80 cm wurde Amberlyst® 35 dry (Kapazität ≥ 5 eq. H⁺/kg) auf eine Füllhöhe von 55 cm eingebracht und die Ionenaustauscherharzkugeln mit zwei Fritten fixiert. Anschließend wurde aus einem Vorlagebehälter 1 über eine Pumpe 2 MNT bei 100°C von unten durch den Ionenaustauscher in einen Auffangbehälter 4 gepumpt. Die Pumpleistung betrug 10 kg/h und die Anfangskonzentration an Natrium betrug vor dem Ionenaustauscher 25 ppm und nach dem Ionenaustauscher konnte kein Natrium nachgewiesen werden. Auf diese Weise wurden 100 kg MNT kontinuierlich behandelt.

### Beispiel 6:

Es wurde genauso verfahren wie im Beispiel 5. Nach der Behandlung von 100 kg MNT wurde das Rohr 3 mit 2 Litern 2 N Schwefelsäure aus Behälter 5 beschickt und ganz gefüllt. Im abgesperrten Zustand wird der Ionentauscher bei 80°C 20 min regeneriert. Danach entleert man das Rohr 3 in einen Auffangbehälter 6 und evakuiert bei 100°C.

Nach dem Trocknen des Ionenaustauschers wurde wieder wie im Beispiel 5 mit Hilfe einer Pumpe MNT bei 100°C durch die Ionenaustauscherkugeln gepumpt. Die Pumpleistung betrug 10 kg/h und die Ausgangskonzentration an Natrium betrug vor dem Ionenaustauscher 25 ppm, nach dem Ionenaustauscher konnte kein Natrium nachgewiesen werden. Auf diese Weise wurden 100 kg MNT kontinuierlich behandelt.

### Beispiel 7:

Es wurde genauso verfahren wie in Beispiel 5 mit der Ausnahme, dass DNT mit einem Nitrokresolat-Anteil von 300 ppm verwendet und die Temperatur auf 80°C begrenzt wurde. Auf diese Weise wurden 100 kg DNT kontinuierlich behandelt. In den 100 kg DNT konnte nach dem Ionenaustauscher kein Natrium nachgewiesen werden.

## Patentansprüche

1. Verfahren zur Verhinderung von Ausfällungen von Nitrohydroxyaromat-Salzen aus den bei der Nitrierung von aromatischen Verbindungen nach alkalischer Wäsche anfallenden nitrierten Rohprodukten, **dadurch gekennzeichnet, dass** man die nitrierten Rohprodukte in Kontakt mit einem sauren Ionentauscher bringt.

2. Verfahren nach Anspruch 1, **dadurch gekennzeichnet, dass** man die nitrierten Rohprodukte über eine Schüttung des sauren Ionentauschers leitet.

3. Verfahren nach Anspruch 1 oder 2, **dadurch gekennzeichnet, dass** die nitrierten Rohprodukte ausgewählt sind unter einem Zulauf zu einer Destillationskolonne, einem Sumpfumlauf einer Destillationskolonne und einem Zulauf zu einem Verdampfer.

4. Verfahren nach einem der vorhergehenden Ansprüche, **dadurch gekennzeichnet, dass** das Inkontaktbringen mit dem sauren Ionentauscher bei einer Temperatur von 0 bis 150°C erfolgt.

5. Verfahren nach einem der vorhergehenden Ansprüche, **dadurch gekennzeichnet, dass** die nitrierten Rohprodukte maximal 0,5 Gew.-% Wasser enthalten.

6. Verfahren nach einem der vorhergehenden Ansprüche, **dadurch gekennzeichnet, dass** die nitrierten Rohprodukte Nitrohydroxyaromat-Natriumsalze enthalten.

7. Verfahren nach einem der vorhergehenden Ansprüche, **dadurch gekennzeichnet, dass** die nitrierten Rohprodukte 0,0001 bis 0,05 Gew.-% Nitrohydroxyaromat-Salze enthalten.

8. Verfahren nach einem der vorhergehenden Ansprüche, **dadurch gekennzeichnet, dass** die nitrierten Rohprodukte wenigstens 85 Gew.-% Mononitrotoluole enthalten.

9. Verfahren nach Anspruch 8, **dadurch gekennzeichnet, dass** die Nitrohydroxyaromat-Salze unter Mononitrokresolaten, Dinitrokresolaten, Trinitrokresolaten und Gemischen davon ausgewählt sind.

10. Produktionsanlage zur Nitrierung nitrierbarer aromatischer Verbindungen, umfassend:
a) eine Nitriereinheit zur Nitrierung nitrierbarer aromatischer Verbindungen,
b) in Produktionslinie stromabwärts zur Nitriereinheit angeordnet, eine Einheit zur Durchführung einer sauren Wäsche mittels Extraktion,
c) in Produktionslinie stromabwärts zur Einheit zur Durchführung der sauren Wäsche angeordnet, eine Einheit zur Durchführung einer alkalischen Wäsche,
d) in Produktionslinie stromabwärts zur Einheit zur Durchführung der alkalischen Wäsche angeordnet, eine Einheit zur Durchführung einer neutralen Wäsche,
e) in Produktionslinie stromabwärts zur Einheit zur Durchführung der neutralen Wäsche angeordnet, eine Destillationseinheit mit einer oder mehreren Destillationskolonnen, und
f) im Zulauf zu wenigstens einer Destillationskolonne und/oder wenigstens einem Sumpfumlauf einer Destillationskolonne, wenigstens eine Ionentauschereinheit zum Inkontaktbringen nitrierter Rohprodukte mit einem sauren Ionentauscher.

## Claims

1. A process for preventing precipitation of nitrohydroxyaromatic salts out of the nitrated crude products obtained in the nitration of aromatic compounds after alkaline scrubbing, which comprises contacting the nitrated crude products with an acidic ion exchanger.

2. The process according to claim 1, wherein the nitrated crude products are passed through a bed of the acidic ion exchanger.

3. The process according to claim 1 or 2, wherein the nitrated crude products are selected from a feed stream to a distillation column, a bottoms circulation stream of a distillation column, and a feed stream to a vaporizer.

4. The process according to any of the preceding claims, wherein the contacting with the acidic ion exchanger is effected at a temperature of 0 to 150°C.

5. The process according to any of the preceding claims, wherein the nitrated crude products comprise not more than 0.5% by weight of water.

6. The process according to any of the preceding claims, wherein the nitrated crude products comprise nitrohydroxyaromatic sodium salts.

7. The process according to any of the preceding claims, wherein the nitrated crude products comprise 0.0001 to 0.05% by weight of nitrohydroxyaromatic salts.

8. The process according to any of the preceding claims, wherein the nitrated crude products comprise at least 85% by weight of mononitrotoluenes.

9. The process according to claim 8, wherein the nitrohydroxyaromatic salts are selected from mononitrocresoxides, dinitrocresoxides, trinitrocresoxides and mixtures thereof.

10. A production plant for nitrating nitratable aromatic compounds, comprising:
a) a nitration unit for nitrating nitratable aromatic compounds,
b) arranged downstream in the production line from the nitration unit, a unit for performing an acidic scrubbing by means of extraction,
c) arranged downstream in the production line from the unit for performing the acidic scrubbing, a unit for performing alkaline scrubbing,
d) arranged downstream in the production line from the unit for performing the alkaline scrubbing, a unit for performing neutral scrubbing,
e) arranged downstream in the production line from the unit for performing the neutral scrubbing, a distillation unit comprising one or more distillation columns, and
f) in the feed to at least one distillation column and/or at least one bottoms circulation system of a distillation column, at least one ion exchanger unit for contacting nitrated crude products with an acidic ion exchanger.

## Revendications

1. Procédé pour empêcher des précipitations de sels de composés hydroxyaromatiques nitrés à partir des produits bruts nitrés formés après lavage alcalin dans la nitration de composés aromatiques, **caractérisé en ce qu'**on met les produits bruts nitrés en contact avec un échangeur d'ions acide.

2. Procédé selon la revendication 1, **caractérisé en ce qu'**on fait passer les produits bruts nitrés audessus d'un lit de l'échangeur d'ions acide.

3. Procédé selon la revendication 1 ou 2, **caractérisé en ce que** les produits bruts nitrés sont choisis parmi un courant d'alimentation arrivant à une colonne de distillation, un courant de recyclage de pied d'une colonne de distillation et un courant d'alimentation arrivant à un évaporateur.

4. Procédé selon l'une quelconque des revendications précédentes, **caractérisé en ce que** la mise en contact avec l'échangeur d'ions acide s'effectue à une température de 0 à 150 °C.

5. Procédé selon l'une quelconque des revendications précédentes, **caractérisé en ce que** les produits bruts nitrés contiennent au maximum 0,5 % en poids d'eau.

6. Procédé selon l'une quelconque des revendications précédentes, **caractérisé en ce que** les produits bruts nitrés contiennent des sels de sodium de composés hydroxyaromatiques nitrés.

7. Procédé selon l'une quelconque des revendications précédentes, **caractérisé en ce que** les produits bruts nitrés contiennent 0,0001 à 0,05 % en poids de sels de composés hydroxyaromatiques nitrés.

8. Procédé selon l'une quelconque des revendications précédentes, **caractérisé en ce que** les produits bruts nitrés contiennent au moins 85 % en poids de mononitrotoluènes.

9. Procédé selon la revendication 8, **caractérisé en ce que** les sels de composés hydroxyaromatiques nitrés sont choisis parmi des mononitrocrésolates, dinitrocrésolates, trinitrocrésolates et des mélanges de ceux-ci.

10. Unité de production pour la nitration de composés aromatiques nitrables, comprenant :
a) une unité de nitration pour la nitration de composés aromatiques nitrables,
b) disposée, dans la ligne de production, en aval de l'unité de nitration, une unité destinée à l'exécution d'un lavage acide par extraction,
c) disposée, dans la ligne de production, en aval de l'unité destinée à l'exécution du lavage acide, une unité destinée à l'exécution d'un lavage alcalin,
d) disposée, dans la ligne de production, en aval de l'unité destinée à l'exécution du lavage alcalin, une unité destinée à l'exécution d'un lavage neutre,
e) disposée, dans la ligne de production, en aval de l'unité destinée à l'exécution du lavage neutre, une unité de distillation comportant une ou plusieurs colonnes de distillation, et
f) dans le courant d'alimentation arrivant à au moins une colonne de distillation et/ou dans au moins un courant de recyclage de pied d'une colonne de distillation, au moins une unité échangeuse d'ions pour la mise en contact de produits bruts nitrés avec un échangeur d'ions acide.
